# EUROPEAN PATENT APPLICATION

(11) **EP 4 112 036 A1**
(43) Date of publication of application: **04.01.2023**
(21) Application number: 21760155.8
(22) Date of filing: 25.02.2021
(51) Int. Cl.: A61K 8/06, A61K 8/02, A61Q 19/00, A61K 8/67

(54) **COMPOSITION HAVING IMPROVED USABILITY**

(30) Priority: 27.02.2020 KR 20200024295; 27.02.2020 KR 20200024296; 27.02.2020 KR 20200024297; 23.02.2021 KR 20210024199
(71) Applicant: Amorepacific Corporation, Seoul 04386 (KR)
(72) Inventor: HWANG, Yoonkyun, Yongin-si, Gyeonggi-do 17074 (KR); AN, Jihye, Yongin-si, Gyeonggi-do 17074 (KR); CHO, Youngsuk, Yongin-si, Gyeonggi-do 17074 (KR); SUH, Byungfhy, Yongin-si, Gyeonggi-do 17074 (KR); CHAE, Byungguen, Yongin-si, Gyeonggi-do 17074 (KR)
(74) Representative: Schön, Christoph
(86) International application number: PCT/KR2021/002404
(87) International publication number: WO 2021/172908

(57) **Abstract**

In one aspect, the present disclosure discloses an emulsion composition and a method for preparing same. The emulsion composition comprises: an inner phase; an external phase; and a plurality of interfacial particles surrounding the surface of the inner phase to form an interface, wherein the interfacial particles have a particle size smaller than the inner phase, and the interfacial particles include organic particles. The method for preparing an emulsion composition comprises the steps of: preparing a colloidal solution comprising a dispersion medium and organic particles dispersed in the dispersion medium; preparing an aqueous phase; preparing an oil phase; and mixing the colloidal solution, the aqueous phase, and the oil phase so as to prepare an emulsion composition comprising an inner phase, an external phase, and a plurality of interfacial particles surrounding the surface of the inner phase to form an interface, wherein the interfacial particles have a particle size smaller than the inner phase, and the interfacial particles include organic particles.

## Description

### [Technical Field]

### [Cross-Reference to Related Application]

The present application claims priority to Korean Patent Application No. 10-2020-0024295 filed on February 27, 2020, Korean Patent Application No. 10-2020-0024296 filed on February 27, 2020, Korean Patent Application No. 10-2020-0024297 filed on February 27, 2020 and Korean Patent Application No. 10-2021-0024199 filed on February 23, 2021 in the Republic of Korea, the disclosures of which are incorporated herein by reference.

The present disclosure relates to an emulsion composition and a method for preparing the same.

### [Background Art]

General emulsification technology includes an emulsification method using a surfactant. Another typically known technology is a Pickering emulsion composition. A Pickering emulsion composition is based on emulsification using inorganic powder. In general, a Pickering emulsion composition ideally uses inorganic powder having a Janus structure with amphipathic property in order to dispose inorganic powder stably at the interface between an aqueous phase and an oil phase. The physical properties of inorganic powder affect the emulsion stability of the emulsion composition. However, there are problems in that it is technically very difficult to carry out emulsification so as to provide such amphipathic properties, production yield is low and high cost is required. For this reason, the Pickering emulsion is hardly applicable to industry. In addition, when inorganic powder does not have suitable oleophilic property, a surfactant is used additionally in order to provide emulsion stability.

An emulsion technology using a polyoxyethylene hydrogenated castor oil derivative is disclosed in the art, wherein polyethylene glycol (PEG) is used to provide both hydrophilicity and oleophilicity so that inorganic powder may be located well at the interface between water and oil. However, it is known that PEG irritates skin's mucous membrane and causes skin troubles or rash. Therefore, with the increased concerned about harmful ingredients of cosmetics, the number of consumers seeking PEG-free cosmetics is increasing and PEG is being excluded from cosmetic materials.

A Pickering emulsion composition uses inorganic powder, and thus shows a disadvantage in that it has a poor moisturizing ability. Thus, the Pickering emulsion composition has a limitation in that it is hardly applicable to products mainly targeting moisturization. Therefore, the Pickering emulsion composition is applied generally to sunscreens using inorganic powder. In addition, the Pickering emulsion composition shows a limitation in terms of its unique finish feeling caused by the inorganic powder particles.

The Pickering emulsion composition shows a disadvantage in terms of formulation stability after freezing and thawing. Since the Pickering emulsion composition uses inorganic powder having amphipathic property instead of a surfactant, it cannot support a functional active ingredient and thus cannot function as a carrier. When porous inorganic powder is used to support an active ingredient, the active ingredient should be coated on the external part of powder. However, when the active ingredient is coated on the external part, it is difficult to ensure that the finished inorganic powder particles have amphipathic property. Therefore, it is required to use an additional surfactant or thickener in order to obtain a stabilized Pickering emulsion composition. As a result, the Pickering emulsion composition has a limitation in that an active ingredient cannot be supported in inorganic powder having amphipathic property and thus it cannot be used as a carrier.

Meanwhile, an active ingredient for improving wrinkles, etc., shows an irritative feeling such as itching, tingling, burning, etc. or causes various inflammatory responses such as erythema, edema, etc., and thus its use is very limited. In addition, there is a problem in that it is not easy to alleviate such skin irritation with a skin irritation-inhibiting material or an anti-inflammatory material.

### [Disclosure]

### [Technical Problem]

In an aspect, the present disclosure is directed to providing an emulsion composition which solves the problems of a Pickering emulsion composition according to the related art.

In another aspect, the present disclosure is directed to providing a method for preparing the emulsion composition.

### [Technical Solution]

In an aspect, the present disclosure provides an emulsion composition including: an inner phase; an outer phase; and a plurality of interfacial particles which surround a surface of the inner phase so as to form an interface, wherein the interfacial particles have a smaller particle size than a size of the inner phase, and the interfacial particles include organic particles.

In another aspect, the present disclosure provides a method for preparing the emulsion composition, which includes preparing a colloidal solution containing a dispersive medium and organic particles dispersed in the dispersive medium; preparing an aqueous phase part; preparing an oil phase part; and mixing the colloidal solution, the aqueous phase part and the oil phase part to prepare an emulsion composition comprising an inner phase, an outer phase and a plurality of interfacial particles which surround a surface of the inner phase so as to form an interface, wherein the interfacial particles have a smaller particle size than a size of the inner phase, and the interfacial particles include organic particles.

### [Advantageous Effects]

In an aspect, the present disclosure provides an emulsion composition which solves the problems of the existing Pickering emulsion composition.

In another aspect, the present disclosure provides a method for preparing the emulsion composition.

### [Brief Description of Drawings]

FIG. 1 shows a result of particle size analysis of nanoemulsion particles according to an exemplary embodiment.
FIG. 2 shows confocal microscopic images showing an inner phase and interfacial particles surrounding the surface of the inner phase of an emulsion composition according to an exemplary embodiment.
FIG. 3 shows a cryo-scanning electron microscopic (Cryo-SEM) image of an emulsion composition according to an exemplary embodiment.
FIG. 4 shows an inner phase and interfacial particles marked on a cryo-scanning electron microscopic (Cryo-SEM) image of an emulsion composition according to an exemplary embodiment.
FIG. 5 shows a cryo-scanning electron microscopic (Cryo-SEM) image of an emulsion composition according to a comparative example.
FIG. 6 shows an optical microscopic image of an emulsion composition according to an exemplary embodiment.
FIG. 7 shows an optical microscopic image of an emulsion composition according to an exemplary embodiment.
FIG. 8 shows an optical microscopic image of an emulsion composition according to a comparative example.
FIG. 9 shows a rheology measurement result of an emulsion composition according to an exemplary embodiment, as compared to conventional O/W and Pickering emulsion compositions. The graph shows the measurement result for a Pickering emulsion composition, a conventional O/W emulsion composition and emulsion compositions of Example 7 and Example 6, sequentially from top to bottom.
FIG. 10 shows a result of investigating the formulation stability of an emulsion composition according to an exemplary embodiment and a conventional O/W emulsion composition after freeze-drying. In the image, the result for the conventional O/W emulsion composition is shown on the left side and the result for the emulsion composition of Example 6 is shown on the right side.
FIG. 11 shows a result of investigating the formulation stability of an emulsion composition according to an exemplary embodiment depending on temperature change. The result of storing the emulsion composition for 4 weeks respectively at room temperature (a), at 37 °C (b), at 45 °C (c), under refrigeration condition (d) and under cycling condition (e) is shown.
FIG. 12 shows a result of comparing the skin delivery effect of an active ingredient according to an exemplary embodiment. The upper image shows the result for an emulsion composition of Example 7 and the lower image shows the result for a conventional O/W emulsion composition.
FIG. 13 shows a result of comparing the skin absorbability of an active ingredient according to an exemplary embodiment. The left bars show the result for the conventional O/W emulsion composition and the right bars show the result for the emulsion composition of Example 7.
FIG. 14 shows a result of investigating the change in skin texture when an emulsion composition according to an exemplary embodiment is applied to skin.
FIG. 15 shows a result of investigating the change in skin water content (%) after application of an emulsion composition according to an exemplary embodiment to skin. The left bars show the result obtained by applying no composition, the central bars show the result obtained by applying a conventional O/W emulsion composition and the right bars show the result obtained by applying an emulsion composition of Example 6.
FIG. 16 shows a result of investigating the change in skin water content under dry environment after application of an emulsion composition according to an exemplary embodiment to skin.
FIG. 17 shows a result of investigating the change in eye rim skin flexibility under dry environment after application of an emulsion composition according to an exemplary embodiment to skin.
FIG. 18 shows a result of investigating the change in eye rim skin elasticity under dry environment after application of an emulsion composition according to an exemplary embodiment to skin.
FIG. 19 shows a result of investigating the change in eye rim wrinkles under dry environment after application of an emulsion composition according to an exemplary embodiment to skin.
FIG. 20 shows a result of investigating the discoloration of retinol contained in an emulsion composition according to an exemplary embodiment. (a) shows an emulsion composition of Example 9 at week 0 and (b) shows the emulsion composition of Example 9 after being stored at 40 °C for 4 weeks.

### [Best Mode]

Hereinafter, the present disclosure is described in detail.

In an aspect, the present disclosure provides an emulsion composition including: an inner phase; an outer phase; and a plurality of interfacial particles which surround a surface of the inner phase so as to form an interface, wherein the interfacial particles have a smaller particle size than a size of the inner phase, and the interfacial particles include organic particles. In the emulsion composition, the inner phase is surrounded by the plurality of interfacial particles which are located on the surface of the inner phase.

In an exemplary embodiment, the inner phase may have a mean size of 1-50 µm. In another exemplary embodiment, the inner phase may have a mean size of 1 µm or larger, 2 µm or larger, 3 µm or larger, 4 µm or larger, 5 µm or larger, 6 µm or larger, 7 µm or larger, 8 µm or larger, 9 µm or larger or 10 µm or larger, and 50 µm or smaller, 45 µm or smaller, 40 µm or smaller, 35 µm or smaller, 30 µm or smaller, 25 µm or smaller, 20 µm or smaller, 15 µm or smaller, 10 µm or smaller or 5 µm or smaller. For example, the inner phase may have a mean size of 5-30 pm, 10-40 µm or 10-30 µm.

In general, an inner phase formed using a surfactant is disadvantageous in that it has a small size of about 1-2 µm and fusion of the inner phase occurs. A Pickering emulsion composition can form an inner phase with a size of about 10-20 µm, but it is difficult to achieve a stable formulation. In an aspect, the emulsion composition according to the present disclosure can form an inner phase having a large size as the Pickering emulsion composition while improving the disadvantage of the Pickering emulsion composition and can prevent the fusion of the inner phase.

In the case of the conventional emulsion composition, the inner phase is generally provided with a small and uniform size in order to improve formulation stability by preventing fusion of the inner phase. In contrast, the emulsion composition according to an aspect of the present disclosure has an advantage in that it shows superior formulation stability even when the inner phase has a large size. In addition, the emulsion composition according to an aspect of the present disclosure exhibits unique rheological property by virtue of the inner phase with a large size, so it can provide customers with a feeling of use different from that of the conventional emulsion composition.

In an exemplary embodiment, the interfacial particles may have a mean particle size corresponding to 1/5,000-1/2 of the mean size of the inner phase. In another exemplary embodiment, the interfacial particles may have a mean particle size of 1/5,000 or larger, 1/4,000 or larger, 1/3,000 or larger, 1/2,000 or larger, 1/1,000 or larger, 1/900 or larger, 1/800 or larger, 1/700 or larger, 1/600 or larger, 1/500 or larger, 1/400 or larger, 1/300 or larger, 1/200 or larger, 1/100 or larger, 1/90 or larger, 1/80 or larger, 1/70 or larger, 1/60 or larger, 1/50 or larger, 1/40 or larger, 1/30 or larger, 1/20 or larger, 1/10 or larger, 1/9 or larger, 1/8 or larger, 1/7 or larger, 1/6 or larger or 1/5 or larger, and 1/2 or smaller, 1/3 or smaller, 1/4 or smaller, 1/5 or smaller, 1/6 or smaller, 1/7 or smaller, 1/8 or smaller, 1/9 or smaller, 1/10 or smaller, 1/20 or smaller, 1/30 or smaller, 1/40 or smaller, 1/50 or smaller, 1/60 or smaller, 1/70 or smaller, 1/80 or smaller, 1/90 or smaller or 1/100 or smaller of the mean size of the inner phase. For example, the interfacial particles may have a mean particle size of 1/3,000-1/30, 1/2,000-1/40 or 1/1,000-1/50 of the mean size of the inner phase when considering manufacturing efficiency or formulation stability.

In an exemplary embodiment, the inner phase may have a mean size of 1-50 µm, and the interfacial particles may have a mean particle size of 1/5,000-1/2 of the mean size of the inner phase.

In an exemplary embodiment, the interfacial particles may have a nanoscale size.

In an exemplary embodiment, the interfacial particles may have a mean particle size of 10 nm or more and less than 1 µm. In another exemplary embodiment, the interfacial particles may have a mean particle size of 10 nm or larger, 50 nm or larger or 100 nm or larger, and smaller than 1 µm, 900 nm or smaller, 800 nm or smaller, 700 nm or smaller, 600 nm or smaller, 500 nm or smaller, 400 nm or smaller, 300 nm or smaller or 200 nm or smaller. For example, the interfacial particles may have a mean particle size of 10-700 nm or 10-500 nm.

In an aspect, the size may refer a diameter.

In an exemplary embodiment, the diameter may mean the longest diameter.

In an exemplary embodiment, the interfacial particles may be continuously located on the surface of the inner phase by being adhered to each other.

In an exemplary embodiment, the interfacial particles may be continuously located on the surface of the inner phase so as to form an interface between the inner phase and the outer phase.

In the emulsion composition according to an aspect of the present disclosure, the interfacial particles may be located on the surface of the inner phase due to the difference in the size of the inner phase and the interfacial particles by controlling the sizes to form an interface between the inner phase and the outer phase. The interfacial particles having a smaller particle size than the inner phase are attracted to the surface of the inner phase, thereby forming an interface between the inner phase and the outer phase. The interfacial particles are adhered to each other due to the attraction between the interfacial particles having a small particle size and surround the surface of the inner phase.

In an exemplary embodiment, the interfacial particles may be used to form an inner phase without additional modification of physical properties. The emulsion composition according to an aspect of the present disclosure does not require the surface treatment of the interfacial particles, e.g., organic particles or a mixture of organic particles and inorganic particles, to have a Janus structure, and allows realization of a stable emulsion system without addition of a surfactant.

In an exemplary embodiment, the interfacial particles may further include inorganic particles.

The emulsion composition according to an aspect of the present disclosure, wherein the organic particles surround the surface of the inner phase to form an interface, does not require surface treatment of the inorganic particles to have a Janus structure unlike the conventional Pickering emulsion composition, and the use of the inorganic particles allows realization of a stable emulsion system without addition of a surfactant.

The inorganic particles refer to particles containing an inorganic material as a main component, wherein the main component refers to the primary component with the largest content in the particles. The content of the main component may be for example, 50 wt% or more, 60 wt% or more, 70 wt% or more, 80 wt% or more or 90 wt% or more.

In an exemplary embodiment, the inorganic particle may be one or more selected from a group consisting of silica, alumina, titania, zirconia, talc, kaolin, mica, sericite, zinc oxide, iron oxide, tin oxide, tungsten oxide, vanadium oxide, copper oxide, calcium carbonate, magnesium carbonate, magnesium silicate, boron nitride, titanated mica and silicon carbide.

In an exemplary embodiment, the inorganic particle may be oleophilic. In an aspect, when the emulsion composition is an O/W formulation, an inorganic particle having oil-dispersible property is dispersible in the inner phase but is not miscible with the outer phase and not dispersible in the outer phase. Since the oil-dispersible inorganic particle is not dispersed in the outer phase, it forms an interface on the surface of the inner phase. In another aspect, when the emulsion composition is a W/O formulation, an inorganic particle having oil-dispersible property is dispersible in the outer phase but is not miscible with the inner phase and not dispersible in the inner phase. Since the oil-dispersible inorganic particle is not dispersed in the inner phase, it forms an interface on the surface of the inner phase.

In an exemplary embodiment, the inorganic particle may be included in an amount of 10 wt% or less based on the total weight of the emulsion composition. In another exemplary embodiment, the inorganic particle may be included in an amount of 0.1 wt% or more or 1 wt% or more, and 10 wt% or less, 9 wt% or less, 8 wt% or less, 7 wt% or less, 6 wt% or less, 5 wt% or less, 4 wt% or less, 3 wt% or less, 2 wt% or less or 1 wt% or less, based on the total weight of the emulsion composition.

In an exemplary embodiment, it may be judged whether the interfacial particles are organic particles or inorganic particles by energy dispersive X-ray spectroscopy (EDS).

EDS (also referred to as EDX or EDAX) is a system capable of analyzing ingredients of a sample when coupled to an electron microscope. It can analyze ingredients of a sample by reacting high-energy electron beams with the sample and then using unique X-ray. Specifically, when electrons generated by applying electric current are accelerated and allowed to collide with a sample, internal electrons present in the sample escape to the outside by the incident electrons to form vacant sites. Then, electrons present in a higher orbital shift to the sites having electron vacancies in order to reduce energy thermodynamically, and thus stabilize the atom. During this process, X-ray energy is emitted by the difference between the two energy orbitals and qualitative analysis of the sample can be performed by measuring the energy. Since all elements have different values of X-ray energy, they can be distinguished from one another by X-ray energy. In addition, it is possible to observe the high-magnification image of the sample by using the electrons (referred to as secondary electrons) escaping to the outside due to the incident electrons.

In an exemplary embodiment, the emulsion composition may include a colloidal solution as an emulsifier. The colloidal solution may contain a dispersive medium and organic particles dispersed in the dispersive medium, the dispersive medium of the colloidal solution may be miscible with the outer phase of the emulsion composition and immiscible with the inner phase of the emulsion composition, and after emulsification with the emulsifier, the dispersive medium may be incorporated into the outer phase, and the organic particles dispersed in the dispersive medium may surround the surface of the inner phase. The emulsifier refers to a substance that enables emulsification. Being added to a mixture of two or more immiscible liquids, e.g., an oil phase and an aqueous phase, so as to mix them, it allows one liquid to be dispersed in another liquid.

In an exemplary embodiment, the dispersive medium may be an aqueous phase, i.e., hydrophilic, and the colloidal solution may be a water-dispersible type. In this case, the emulsion composition containing the water-dispersible colloidal solution becomes an O/W formulation, and the organic particles have water-dispersible property. In an exemplary embodiment, when the emulsion composition is an O/W formulation, the organic particles may have a hydrophilic surface. For example, the entire surface of the organic particles may be hydrophilic.

In an exemplary embodiment, the dispersive medium may be an oil phase, i.e., oleophilic, and the colloidal solution may be an oil-dispersible type. In this case, the emulsion composition containing the oil-dispersible colloidal solution may be a W/O formulation, and the organic particles have oil-dispersible property. In an exemplary embodiment, when the emulsion composition is a W/O formulation, the organic particles may have an oleophilic surface. For example, the entire surface of the organic particles may be oleophilic.

In an exemplary embodiment, the organic particles may be dispersible in the dispersive medium of the colloidal solution. In another exemplary embodiment, the organic particles may be dispersible in the outer phase of the emulsion composition but not in the inner phase, unlike the emulsion composition using a surfactant having a property of being dispersed in both inner and outer phases or the Pickering emulsion composition using particles of a Janus structure. In an aspect, when the emulsion composition is an O/W formulation, the organic particles are added to the emulsion composition after being dispersed in a water-dispersible colloidal solution. Accordingly, since the organic particles having water-dispersible property are dispersible in the outer phase of the emulsion composition but not in the inner phase, they form an interface on the surface of the inner phase. In another aspect, when the emulsion composition is a W/O formulation, the organic particles are added to the emulsion composition after being dispersed in an oil-dispersible colloidal solution. Accordingly, since the organic particles having oil-dispersible property are dispersible in the outer phase of the emulsion composition but not in the inner phase, they form an interface on the surface of the inner phase.

As mentioned above, the dispersive medium of the colloidal solution contained in the emulsion composition according to an aspect of the present disclosure is miscible with the outer phase of the emulsion composition and immiscible with the inner phase of the emulsion composition. If the organic particles dispersed in the dispersive medium are dispersed only in the outer phase of the emulsion composition, not in the inner phase, the organic particles are not mixed in the inner phase of the emulsion composition and are located continuously on the surface of the inner phase, thereby forming an interface. In other words, the organic particles that do not have dispersibility for the inner phase of the emulsion composition are not mixed in the inner phase and surround the surface of the inner phase. If the dispersive medium of the colloidal solution is miscible with the inner phase of the emulsion composition, an interface cannot be formed between the inner phase and the outer phase since the organic particles dispersed in the dispersive medium are mixed in the inner phase. In addition, if the organic particles are not dispersed in the outer phase, an interface cannot be formed in such a manner that the organic particles are attracted to the inner phase and surround the surface of the inner phase.

The emulsion composition according to an aspect of the present disclosure is emulsified by dispersing the organic particles in the outer phase and stabilizing the inner phase with the organic particles. An interface may be formed on the surface of the inner phase as the organic particles dispersed in the outer phase are attracted to the inner phase having a larger size.

In an exemplary embodiment, the emulsion composition may include a colloidal solution as an emulsifier, the colloidal solution may contain a dispersive medium and organic particles dispersed in the dispersive medium, the dispersive medium of the colloidal solution may be miscible with the outer phase of the emulsion composition and immiscible with the inner phase of the emulsion composition, and after emulsification with the emulsifier, the dispersive medium may be incorporated into the outer phase, and the organic particles dispersed in the dispersive medium may surround the surface of the inner phase. The organic particles may be dispersible in the outer phase of the emulsion composition but not in the inner phase.

In an exemplary embodiment, the colloidal solution may be contained in an amount of 7-30 wt% based on the total weight of the emulsion composition. In another exemplary embodiment, the colloidal solution may be contained in an amount of 7 wt% or more, 9 wt% or more, 11 wt% or more, 13 wt% or more, 15 wt% or more, 17 wt% or more, 19 wt% or more, 21 wt% or more, 23 wt% or more, 25 wt% or more or 27 wt% or more, and 30 wt% or less, 28 wt% or less, 26 wt% or less, 24 wt% or less, 22 wt% or less, 20 wt% or less, 18 wt% or less, 16 wt% or less, 14 wt% or less, 12 wt% or less or 10 wt% or less, based on the total weight of the emulsion composition.

In an exemplary embodiment, the organic particles may be present at a weight ratio of 0.1-5 times based on the total weight of the inner phase. When the emulsion composition is an O/W formulation, the total weight of the inner phase means the total weight of the oil phase part. And, when the emulsion composition is a W/O formulation, the total weight of the inner phase means the total weight of the aqueous phase part. In another exemplary embodiment, the organic particles may be present at a weight ratio of 0.1 time or larger, 0.2 time or larger, 0.3 time or larger, 0.4 time or larger, 0.5 time or larger, 0.6 time or larger, 0.7 time or larger, 0.8 time or larger, 0.9 time or larger, 1 time or larger, 1.1 times or larger, 1.2 times or larger, 1.3 times or larger, 1.4 times or larger, 1.5 times or larger, 1.6 times or larger, 1.7 times or larger, 1.8 times or larger, 1.9 times or larger or 2 times or larger, and 5 times or smaller, 4.5 times or smaller, 4 times or smaller, 3.5 times or smaller, 3 times or smaller, 2.5 times or smaller, 2 times or smaller, 1,5 times or smaller or 1 time or smaller, based on the total weight of the inner phase. For example, the organic particles may be specifically present at a weight ratio of 0.4-3 times or 1.2-3 times based on the total weight of the inner phase when considering formulation stability.

In an exemplary embodiment, the colloidal solution may be contained in an amount of 7-30 wt% based on the total weight of the emulsion composition, and the organic particles may be present at a weight ratio of 0.1-5 times based on the total weight of the inner phase.

The organic particles such as nanoemulsion particles, solid lipid nanoparticles, liposomes, polymersomes, etc., which have been used only as a carrier for an active ingredient, have been added to an emulsion composition only in a small amount and the organic particles are not located on the surface of the inner phase of the emulsion composition. In contrast, the emulsion composition according to an aspect of the present disclosure is different from the emulsion composition containing conventional organic particles in that it contains the organic particles at a higher content and the organic particles are located on the surface of the inner phase of the emulsion composition.

The organic particles refer to particles containing an organic material as a main component, wherein the main component refers to the primary component with the largest content in the particles. The content of the main component may be for example, 50 wt% or more, 60 wt% or more, 70 wt% or more, 80 wt% or more or 90 wt% or more.

In an exemplary embodiment, the organic particles may be formed from a lipid.

In an exemplary embodiment, the organic particles may include a lipid.

In an exemplary embodiment, the lipid may include one or more selected from a group consisting of a phospholipid, a wax, butter and a ceramide.

In an exemplary embodiment, the phospholipid may be one or more selected from a group consisting of hydrogenated lecithin, hydrogenated phosphatidylcholine, soybean phospholipid, hydrogenated lysophosphatidylcholine, hydrogenated lysolecithin and unsaturated lecithin.

In an exemplary embodiment, the wax may be one or more selected from a group consisting of vegetable wax, animal wax, mineral wax and synthetic wax.

In an exemplary embodiment, the wax may be one or more selected from a group consisting of candelilla wax, carnauba wax, ozokerite, ceresin wax, montane wax, microcrystalline wax, tribehenin, glyceryl behenate, glyceryl dibehenate, glyceryl tribehenate, stearyl behenate and trihydroxystearin.

In an exemplary embodiment, the butter may be one or more selected from a group consisting of shea butter, cocoa butter, almond butter, apricot butter, peach butter, cupuasu butter, pistachio butter, olive butter, aloe butter, vanilla butter, illiff butter, camellia butter, babassu butter, avocado butter, jojoba butter, kokum butter, cacao butter, mango butter, bean butter, grapeseed butter, murumuru butter and macadamia seed butter.

In an exemplary embodiment, the ceramide may be a natural ceramide, a synthetic ceramide or a pseudoceramide.

The pseudoceramide is a synthetic material which is structurally similar to a natural ceramide and has properties similar to those of natural ceramides such as skin protection function, water holding ability, etc. For example, the pseudoceramide may be hydroxypropyl bispalmitamide MEA.

In an exemplary embodiment, the organic particles may be selected from a group consisting of nanoemulsion particles, solid lipid nanoparticles (SLN), liposomes and polymersomes. The nanoemulsion particles, solid lipid nanoparticles, liposomes or polymersomes may be prepared according to common preparation methods known in the art.

In general, emulsions can be classified into three types, a microemulsion, a nanoemulsion and a macroemulsion depending on the average size of the inner phase. The nanoemulsion refers to one in which one liquid is dispersed in the form of small particles in another in two or more liquids immiscible with each other, and the size of the particles is a nanoscale size. The nanoemulsion particles mean the inner phase of the nanoemulsion, that is, emulsion particles.

The solid lipid nanoparticles are known as one of the drug delivery systems suggested to overcome the disadvantages of the conventional colloidal carriers. The size of the solid lipid nanoparticles may be determined by various factors, such as types and amounts of lipids used for the solid lipid nanoparticles, types and amounts of surfactants, or the like.

The liposome is a spherical or elliptical structure formed from a lipid. It is characterized in that it has an inner space isolated from the outside by at least one bilayer membrane. For example, a spontaneously aligned bilayer membrane structure may be formed by the interaction of molecules having hydrophobicity and hydrophilicity at the same time, such as phospholipids.

The polymersome has a structure similar to that of a liposome. It has a membrane surrounding the internal fluid, and the membrane may include a polymer. For example, a molecular bilayer membrane structure may be formed by self-assembly of amphipathic copolymers similar to phospholipids.

In an exemplary embodiment, the organic particle may include an active ingredient.

The active ingredient may be, for example, an active ingredient that can be dissolved in oil, such as vitamin A, e.g., retinol, vitamin E, carotene, coenzyme Q₁₀, resveratrol, beta-carotene, bakuchiol, lycopene, etc.

In an exemplary embodiment, the active ingredient may include bakuchiol and/or retinol.

In an exemplary embodiment, the organic particles may no differentiation between an inner layer and an outer layer or may have a layered structure with two or more layers.

In an exemplary embodiment, the active ingredient may be supported on the organic particles. That is to say, when the organic particles have a layered structure with two or more layers, an active ingredient for whitening, anti-wrinkling, anti-oxidation, etc. may be support. In an exemplary embodiment, the organic particles may support a water-soluble or oil-soluble active ingredient.

The emulsion composition according to an aspect of the present disclosure provides the effect of increasing the absorption or delivery of the active ingredient to skin. In addition, since the emulsion composition according to an aspect of the present disclosure enables superior absorption or delivery of the active ingredient to skin, the desired effect can be achieved only with a small amount of the active ingredient.

In an exemplary embodiment, the emulsion composition may reduce skin irritation caused by the active ingredient. In another exemplary embodiment, the organic particles may reduce skin irritation caused by the active ingredient in the emulsion composition. As the content of the active ingredient, e.g., for whitening, anti-wrinkling, anti-oxidation, etc. in the composition is increased, it shows a more advantageous effect. However, since skin irritation occurs as the content of the active ingredient is increased, there is a limitation in that the active ingredient should be used at a low content in the industrial field. In an aspect, the emulsion composition according to the present disclosure overcomes the limitation of a low content of the active ingredient according to the related art, and allows the emulsion composition to contain an increased content of the active ingredient with reduced skin irritation.

In an exemplary embodiment, the active ingredient may be present in an amount of 0.0001-10 wt% based on the total weight of the emulsion composition. In another exemplary embodiment, the active ingredient may be present in an amount of 0.0001 wt% or more, 0.001 wt% or more, 0.01 wt% or more, 0.1 wt% or more, 1 wt% or more or 2 wt% or more, and 10 wt% or less, 9 wt% or less, 8 wt% or less, 7 wt% or less, 6 wt% or less, 5 wt% or less, 4 wt% or less, 3 wt% or less or 2 wt% or less, based on the total weight of the emulsion composition.

In an exemplary embodiment, the interfacial particles may have an amorphous, spherical or elliptical shape.

In an exemplary embodiment, the components and/or size of the interfacial particles may be designed variously depending on the feeling of use, effect, etc. of the emulsion composition.

In an exemplary embodiment, the emulsion composition may be a cosmetic composition. In an exemplary embodiment, the cosmetic composition may be a formulation in the form of a mist, a spray, a toner, an essence, a gel, a lotion, a cream, a pack, a foam cleanser, a makeup base, a foundation, etc.

In an exemplary embodiment, the emulsion composition may be a cosmetic composition with the skin irritation by the active ingredient reduced.

In an exemplary embodiment, the emulsion composition may be a composition for external application to skin. The composition for external application to skin refers to a composition applied from outside of skin, and may include various type of medical formulations. In an exemplary embodiment, the composition for external application to skin may be a formulation in the form of a mist, a spray, a suspension, an emulsion, a gel, a lotion, an ointment, etc.

In an exemplary embodiment, the emulsion composition may not contain a surfactant. The surfactant refers to a substance having two different properies which allow two or more immiscible liquids, e.g., an oil phase and an aqueous phase, to be mixed with each other.

In an exemplary embodiment, the emulsion composition may further contain a surfactant.

In an exemplary embodiment, when the emulsion composition contains a surfactant, the inner phase may have a mean size of 1-10 µm.

In an exemplary embodiment, the emulsion composition may not contain a thickener.

In an exemplary embodiment, the emulsion composition may further contain a thickener.

In an exemplary embodiment, the emulsion composition may contain neither a surfactant nor a thickener.

In an exemplary embodiment, the emulsion composition may further contain a surfactant and a thickener.

The emulsion composition according to an aspect of the present disclosure may be prepared into various formulations ranging from a low-viscosity essence or lotion to a high-viscosity cream by using no thickener or using various types of thickeners.

In an exemplary embodiment, the emulsion composition may be an oil-in-water (O/W) formulation. The emulsion composition according to an aspect of the present disclosure can be prepared using interfacial particles and exhibits superior formulation stability regardless of the type or polarity of oil. In other words, the inner phase can be formed without being affected by the oil phase.

In an exemplary embodiment, the emulsion composition may exhibit a yield stress of 0.1-1.0 Pa when viscosity depending on shear stress is measured.

In an exemplary embodiment, the viscosity may be measured depending on shear stress using a rheometer under the condition of osc.stress (Pa) 0.1-1000 @ frequency 50 Hz and room temperature.

In an exemplary embodiment, the emulsion composition may undergo decrease in viscosity by 30% or more, 40% or more or 50% or more out of the yield stress of 0.1-1.0 Pa.

The emulsion composition having the above-defined range of yield stress according to an aspect of the present disclosure provides a unique feeling of use such as initial crumbling feeling, i.e. initial water burst feeling. Such feeling of use may be varied by controlling the content of the interfacial particles.

In an exemplary embodiment, the emulsion composition may exhibit an interval in which viscosity is increased as shear stress is increased, in at least a part of a shear stress interval of 100-1,000 Pa, 100-700 Pa, or 100-500 Pa, when viscosity depending on shear stress is measured.

In an exemplary embodiment, the viscosity may be measured depending on shear stress using a rheometer under the condition of osc.stress (Pa) 0.1-1000 @ frequency 50 Hz and room temperature.

In an exemplary embodiment, the emulsion composition may exhibit a yield stress of 0.1-1.0 Pa, a decrease in viscosity by 30% or more, 40% or more or 50% or more out of the yield stress of 0.1-1.0 Pa and an interval in which viscosity is increased as shear stress is increased, in at least a part of a shear stress interval of 100-1,000 Pa, when viscosity depending on shear stress is measured.

The emulsion composition according to an aspect of the present disclosure provides smooth finish feeling and improved skin roughness and moisturization derived from the interfacial particles in later stage upon application to skin. The interfacial particles present at the interface between the inner phase and the outer phase are packed on the skin surface to improve skin roughness, while inhibiting evaporation of water by virtue of an occlusive effect to retain a high skin water content.

In an exemplary embodiment, the emulsion composition may be in the form of a freeze-dried formulation. The freeze-dried formulation retains stability even during long-term storage and provides high portability and transportability because it contains no liquid.

In an exemplary embodiment, the emulsion composition may cause no oil separation.

In an exemplary embodiment, the emulsion composition may cause no oil separation upon freeze-drying.

In an exemplary embodiment, the oil separation may be observable with naked eyes.

In an exemplary embodiment, the freeze-drying may be performed by freezing at -60 °C or lower or at -120 to -60 °C and then subliming and removing water under reduced pressure.

In an exemplary embodiment, the freeze-drying may be performed at a temperature of -120 to -60 °C, -120 to -100 °C or -120 to -110 °C.

In an exemplary embodiment, the freeze-drying may be performed at a pressure of 1-100 mTorr, 1-50 mTorr, 10-80 mTorr or 20-40 mTorr.

In an exemplary embodiment, the freeze-drying may be performed at a temperature of -120 to -60 °C and at a pressure of 1-100 mTorr.

In another aspect, the present disclosure provides a method for preparing the emulsion composition, which includes: preparing a colloidal solution containing a dispersive medium and organic particles dispersed in the dispersive medium; preparing an aqueous phase part; preparing an oil phase part; and mixing the colloidal solution, the aqueous phase part and the oil phase part to prepare an emulsion composition containing an inner phase, an outer phase and a plurality of interfacial particles which surround a surface of the inner phase so as to form an interface, wherein the interfacial particles have a smaller particle size than a size of the inner phase, and the interfacial particles include organic particles.

In an exemplary embodiment, the method may be an emulsification method.

In the method described above, the sequence of preparation and/or mixing of the colloidal solution, the aqueous phase part and the oil phase part may be adequately selected by those skilled in the art.

In an exemplary embodiment, after mixing the colloidal solution and the aqueous phase part, the mixture may be mixed with the oil phase part. In another exemplary embodiment, after mixing the colloidal solution and the oil phase part, the mixture may be mixed with the aqueous phase part. In another exemplary embodiment, after mixing the aqueous phase part and the oil phase part, the mixture may be mixed with the colloidal solution. In another exemplary embodiment, the colloidal solution, the aqueous phase part and the oil phase part may be mixed at once.

In an exemplary embodiment, for preparation of an O/W emulsion composition, after adding a water-dispersible colloidal solution to the aqueous phase part, the mixture may be mixed with the oil phase part.

In an exemplary embodiment, for preparation of a W/O emulsion composition, after adding an oil-dispersible colloidal solution to the oil phase part, the mixture may be mixed with the aqueous phase part.

In an exemplary embodiment, the organic particles dispersed in the outer phase and having a smaller particle size as compared to the inner phase may be attracted to the inner phase, and an interface may be formed on the surface of the inner phase as they are not mixed with the inner phase.

In an exemplary embodiment, the method may further include adding inorganic particles to the oil phase part. For example, the method may further include, after adding inorganic particles to the oil phase part to prepare an oil phase part, mixing the same with the colloidal solution, the aqueous phase part or a mixture of the colloidal solution and the aqueous phase part.

In an exemplary embodiment, the method may further include adding inorganic particles to the emulsion composition. For examples, inorganic particles may be added to the emulsion composition which has been emulsified.

In an aspect, the present disclosure includes at least the following embodiments.

Embodiment 1. An emulsion composition including: an inner phase; an outer phase; and a plurality of interfacial particles which surround a surface of the inner phase so as to form an interface, wherein the interfacial particles have a smaller particle size than a size of the inner phase, and the interfacial particles include organic particles.

Embodiment 2. The emulsion composition according to Embodiment 1, wherein the inner phase has a mean size of 1-50 µm.

Embodiment 3. The emulsion composition according to Embodiment 1 or 2, wherein the interfacial particles have a mean particle size corresponding to 1/5,000-1/2 of the mean size of the inner phase.

Embodiment 4. The emulsion composition according to any of Embodiments 1 to 3, wherein the interfacial particles have a mean particle size of 10 nm or more and less than 1 µm.

Embodiment 5. The emulsion composition according to any of Embodiments 1 to 4, wherein the interfacial particles are continuously located on the surface of the inner phase to form the interface between the inner phase and the outer phase.

Embodiment 6. The emulsion composition according to any of Embodiments 1 to 5, wherein the interfacial particles further include inorganic particles.

Embodiment 7. The emulsion composition according to any of Embodiments 1 to 6, wherein the emulsion composition contains a colloidal solution as an emulsifier, the colloidal solution contains a dispersive medium and organic particles dispersed in the dispersive medium, the dispersive medium of the colloidal solution is miscible with the outer phase of the emulsion composition and immiscible with the inner phase of the emulsion composition, and after emulsification with the emulsifier, the dispersive medium is incorporated into the outer phase, and the organic particles dispersed in the dispersive medium surround the surface of the inner phase.

Embodiment 8. The emulsion composition according to Embodiment 7, wherein the colloidal solution is present in an amount of 7-30 wt% based on the total weight of the emulsion composition.

Embodiment 9. The emulsion composition according to any of Embodiments 1 to 8, wherein the organic particles are dispersed in the outer phase of the emulsion composition and not in the inner phase of the emulsion composition.

Embodiment 10. The emulsion composition according to any of Embodiments 1 to 9, wherein the organic particles are selected from a group consisting of nanoemulsion particles, solid lipid nanoparticles (SLN), liposomes and polymersomes.

Embodiment 11. The emulsion composition according to any of Embodiments 1 to 10, wherein the organic particles include an active ingredient.

Embodiment 12. The emulsion composition according to Embodiment 11, wherein the active ingredient includes bakuchiol or retinol.

Embodiment 13. The emulsion composition according to Embodiment 11 or 12, wherein the active ingredient is supported on the organic particles.

Embodiment 14. The emulsion composition according to any of Embodiments 11 to 13, wherein the active ingredient is present in an amount of 0.0001-10 wt% based on the total weight of the emulsion composition.

Embodiment 15. The emulsion composition according to any of Embodiments 1 to 14, wherein the emulsion composition is a cosmetic composition.

Embodiment 16. The emulsion composition according to any of Embodiments 1 to 15, wherein the emulsion composition further contains a surfactant.

Embodiment 17. The emulsion composition according to any of Embodiments 1 to 16, wherein the emulsion composition further contains a thickener.

Embodiment 18. The emulsion composition according to any of Embodiments 1 to 17, wherein the emulsion composition exhibits a yield stress of 0.1-1.0 Pa when viscosity depending on shear stress is measured.

Embodiment 19. The emulsion composition according to Embodiment 18, wherein the emulsion composition exhibits a decrease in viscosity by 30% or more out of the yield stress of 0.1-1.0 Pa.

Embodiment 20. The emulsion composition according to any of Embodiments 1 to 19, wherein the emulsion composition exhibits an interval in which viscosity is increased as shear stress is increased, in at least a part of a shear stress interval of 100-1,000 Pa, when viscosity depending on shear stress is measured.

Embodiment 21. The emulsion composition according to any of Embodiments 1 to 20, wherein the emulsion composition is a freeze-dried formulation.

Embodiment 22. A method for preparing the emulsion composition according to any of Embodiments 1 to 21, including: preparing a colloidal solution containing a dispersive medium and organic particles dispersed in the dispersive medium; preparing an aqueous phase part; preparing an oil phase part; and mixing the colloidal solution, the aqueous phase part and the oil phase part to prepare an emulsion composition including an inner phase, an outer phase and a plurality of interfacial particles which surround a surface of the inner phase so as to form an interface, wherein the interfacial particles have a smaller particle size than a size of the inner phase, and the interfacial particles comprise organic particles.

Embodiment 23. The method according to Embodiment 22, which further comprises adding inorganic particles to the oil phase part or the emulsion composition.

### [Mode for Invention]

Hereinafter, the present disclosure will be explained in more detail with reference to examples. The following examples are for illustrative purposes only. In addition, it will be apparent to those skilled in the art that the scope of the present disclosure is not limited to the examples.

### Preparation Examples: Preparation of colloidal solutions

Water-dispersible colloidal solutions containing nanoemulsion particles as organic particles were prepared according to the composition (wt%) described in Table 1. The oil phase part was heated and dissolved at 70 °C and dispersed by a homogenizer to obtain an oleophilic mixture. In a separate container, the aqueous phase part was heated and dissolved at 70 °C, and the oleophilic mixture obtained as mentioned above was added gradually to the aqueous phase part, thereby forming water-dispersible organic nanoparticles at 70 °C through a homogenizer. An ultrasonic high-pressure homogenizer was used as a homogenizer to control the size of nanoparticles.

**[Table 1]**

| | Prep. Ex. 1 | Prep. Ex. 2 | Prep. Ex. 3 | Prep. Ex. 4 |
|---|---|---|---|---|
| Water | To 100 | To 100 | To 100 | To 100 |
| 1,2-Hexanediol | 1 | 1 | 1 | 1 |
| Butylene glycol | 10 | 10 | 10 | 10 |
| Glycerin | | 5 | 5 | 5 |
| Soybean oil * retinol * BHT (dibutylhydroxytoluene) | | | 10-30 | |
| Bakuchiol | | | | 5-25 |
| Ceramide 3 | 1 | 1 | 1 | 1 |
| Cholesterol | 1 | 1 | 1 | 1 |
| Stearic acid * Palmitic acid | 0.8 | 0.8 | 0.8 | 0.8 |
| Tocopherol | 0.5 | 5 | 5 | 5 |
| Phytosteryl/octyldodecyl lauroyl glutamate | 1 | 1 | 1 | 1 |
| Shea butter | 3 | 6 | 6 | 6 |
| Hydrogenated lecithin | 3 | 3 | 3 | 3 |
| Meadowfoam seed oil | 6 | 2 | 2 | 2 |

In addition, a water-dispersible colloidal solution containing solid lipid nanoparticles was prepared according to the composition (wt%) described in Table 2. The oil phase part was heated and dissolved at 70 °C and dispersed by a homogenizer to obtain an oleophilic mixture. In a separate container, the aqueous phase part was heated and dissolved at 70 °C, and the oleophilic mixture obtained as mentioned above was added gradually to the aqueous phase part, thereby forming water-dispersible organic nanoparticles at 70 °C through a homogenizer. When the resultant product was cooled, recrystallization of lipid occurred and organic nanoparticles were formed. An ultrasonic high-pressure homogenizer was used as a homogenizer to control the size of nanoparticles.

**[Table 2]**

| | Preparation Example 5 |
|---|---|
| Water | To 100 |
| 1,2-Hexanediol | 1 |
| Ethylhexylglycerin | 0.05 |
| Butylene glycol | 20 |
| Hydroxypropyl bispalmitamide MEA | 1 |
| Polyglyceryl-3 methylglucose distearate | 7 |
| Hydrogenated lecithin | 1 |
| Stearyl behenate | 13 |

In addition, a water-dispersible colloidal solution containing liposomes as organic particles was prepared according to the composition described in Table 3. 100% hydrogenated oleoyl-palmitoyl/oleoyl-stearyl phosphatidylcholine mixture (Lipoid S100-3) and cholesterol were dissolved in ethanol under warming to obtain a mixed solution. The mixed solution was introduced to water at 60 °C and mixed and agitated with a homogenizer at a speed of 5000 rpm for 5 minutes. Then, a high-pressure homogenizer (1000 bar, 3 cycles) was used to obtain water-dispersible organic nanoparticles. A rotary evaporator was used to remove the residual ethanol solution, thereby providing liposomes including lipid-cholesterol.

**[Table 3]**

| | Preparation Example 6 (g) |
|---|---|
| Cholesterol | 0.67 |
| Oleoyl-palmitoyl/oleoyl-stearyl phosphatidylcholine mixture (Lipoid S100-3) | 5.33 |
| Ethanol | 30 |
| Water | 300 |
| Total weight after distillation | 100 |

Further, a water-dispersible colloidal solution containing polymersomes as organic particles was prepared according to the composition described in Table 4. Poly(methacrylic acid-co-stearyl methacrylate) copolymer, 100% hydrogenated oleoyl-palmitoyl/oleoyl-stearyl phosphatidylcholine mixture (Lipoid S100-3) and cholesterol were dissolved in ethanol under warming to obtain a mixed solution. The mixed solution was introduced to water at 60 °C and mixed and agitated with a homogenizer at a speed of 5000 rpm for 5 minutes to obtain primarily dispersed polymer-liposome complexes. Then, a high-pressure homogenizer (1000 bar, 3 cycles) was used to obtain water-dispersible organic nanoparticles. A rotary evaporator was used to remove the residual ethanol solution, thereby providing polymer-liposome nanocomplexes using an acidity-sensitive polymer.

**[Table 4]**

| | Preparation Example 7 (g) |
|---|---|
| Poly(methacrylic acid-co-stearyl methacrylate) copolymer | 1.8 |
| Oleoyl-palmitoyl/oleoyl-stearyl phosphatidylcholine mixture (Lipoid S100-3) | 3.15 |
| Cholesterol | 1.05 |
| Ethanol | 30 |
| Water | 300 |
| Total weight after distillation | 100 |

### Examples: Preparation of emulsion compositions

Emulsion compositions were prepared using the water-dispersible colloidal solutions prepared in Preparation Examples 1 and 5 as an emulsifier. The emulsion compositions were prepared using organic nanoparticles dispersed in the water-dispersible colloidal solution as interfacial particles or using inorganic particles and organic nanoparticles dispersed in the water-dispersible colloidal solutions as interfacial particles. In addition, an emulsion composition of Comparative Example 1 was prepared using a surfactant as an emulsifier. The emulsion compositions were prepared with the compositions (wt%) descried in Table 5.
1) The aqueous phase part was warmed to 50-75 °C to mix and dissolve the ingredients. The water-dispersible colloidal solutions were added to the aqueous phase part.
2) The remaining oil phase part was warmed to 50-75 °C to mix and dissolve the ingredients. The inorganic particles were added to the oil phase part.
3) The aqueous phase part was mixed with the oil phase part, while maintaining the temperature at 50-75 °C, and O/W emulsion compositions were prepared by using a homogenizer.

**[Table 5]**

| | Ex.1 | Ex.2 | Ex.3 | Ex.4 | Ex.5 | Ex.6 | Ex.7 | Comp. Ex.1 |
|---|---|---|---|---|---|---|---|---|
| Hydrogenated C₆₋₁₄ olefin polymer | 3 | 3 | 3 | 3 | 3 | 10 | 10 | 3 |
| Squalane | 2 | 2 | 2 | 2 | 2 | | | 2 |
| *Helianthus annuus* (sunflower) seed oil | | | | | | 3 | 3 | |
| Cyclopentasiloxane * cyclohexasiloxane | 5 | 5 | 5 | 5 | 5 | 2 | 2 | 5 |
| Diisostearoyl polyglyceryl-3 dimer dilinoleate | | | | | | | | 1.5 |
| Polyglyceryl-4 diisostearate/polyhydroxyste arate/sebacate | | | | | | | | 1.5 |
| Shea butter | 1.5 | 1.5 | 1.5 | 1.5 | 1.5 | | | 1.5 |
| Glyceryl stearate * PEG-100 stearate | | | | 0.5 | | | | |
| Titanium dioxide (nano) * stearic acid * alumina | 1 | 3 | 3 | | | | | |
| Water | To 100 | To 100 | To 100 | To 100 | To 100 | To 100 | To 100 | To 100 |
| Water-dispersible colloidal solution of Preparation Example 1 | 9 | 7 | 7 | 9.5 | 10 | | 10 | |
| Water-dispersible colloidal solution of Preparation Example 5 | | | | | | 10 | | |
| 1,2-Hexanediol | 1 | 1 | 1 | 1 | 1 | 1 | 1 | 1 |
| Ethylhexylglycerin | 0.05 | 0.05 | 0.05 | 0.05 | 0.05 | 0.05 | 0.05 | 0.05 |
| Polyacrylate-13 * polyisobutene * water/aqua/EAU * polysorbate 20 * sorbitan isostearate | | | 1 | | | | | |
| Carbomer | 0.2 | 0.2 | | 0.2 | 0.2 | 0.2 | 0.2 | 0.2 |
| Tromethamine | 0.1 | 0.1 | | 0.1 | 0.1 | 0.1 | 0.1 | 0.1 |

Whereas the emulsion compositions of Examples 1-7 showed high emulsion stability, the emulsion composition of Comparative Example 1 prepared by adding diisostearoyl polyglyceryl-3 dimer dilinoleate and polyglyceryl-4 diisostearate/polyhydroxystearate/sebacate as surfactants showed low emulsion stability. In the emulsion compositions of Examples 1-7, the interfacial particles formed an interface between the inner phase and the outer phase and the inner phase was stable.

The emulsion compositions of Examples 1-3 contain not only organic particles but also inorganic particles as the interfacial particles. In general, when inorganic particles are contained in an emulsion system, the stability of the emulsion system is reduced. Thus, it is required to use a surfactant in order to supplement this. In contrast, it was confirmed that the emulsion compositions according to an aspect of the present disclosure maintain emulsion stability even when they include inorganic particles, and thus form stable emulsion systems without using any surfactant. The emulsion systems maintained stability even when the content of inorganic particles was increased. In addition, the thickener did not affect the interfacial stability between the inner phase and the outer phase. Therefore, it was confirmed that the emulsion compositions according to an aspect of the present disclosure can maintain the stability of the emulsion systems without being affected by the type of thickener.

The emulsion compositions of Examples 4-7 use only organic particles as interfacial particles and the emulsion composition of Example 4 further contains a surfactant. Like the emulsion compositions of Examples 1-3, it was confirmed that the emulsion compositions of Examples 4-7 form stable emulsion systems. In addition, it was confirmed that the emulsion stability can be improved further by adding the surfactant in addition to the interfacial particles. A superior emulsion stability could be provided even with a very small amount as compared to the content of the surfactant generally used for forming emulsion compositions. Skin irritation could be minimized since the amount of the surfactant could be reduced. Meanwhile, the addition of the surfactant resulted in decreased size of the inner phase.

The emulsion composition of Comparative Example 1 was emulsified with a surfactant without using interfacial particles. The emulsion composition of Comparative Example 1 showed fusion of the inner phase with the lapse of time and showed oil separation after freeze-drying, and thus exhibited reduced formulation stability as compared to the emulsion compositions of Examples 1-7.

### Test Example 1. Particle size analysis of interfacial particles

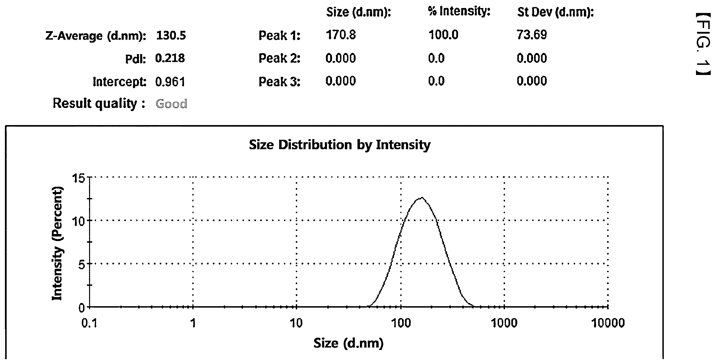

The nanoemulsion particles prepared in Preparation Example 1 were analyzed by using a particle size analyzer (Mastersizer, Malvern, UK). As a result, it was found out that the nanoemulsion particles have an average particle size of 130.5 nm (see, FIG. 1). PDI is a measure of heterogeneity index. A higher PDI value means a dispersion state with higher heterogeneity. It was confirmed that the nanoemulsion particles prepared in Preparation Example 1 have a relatively stable and homogeneous dispersion state.

### Test Example 2. Confirmation of inner phase of emulsion composition

For investigation of the structure of the inner phase of the emulsion composition, a water-dispersible colloidal solution containing solid lipid nanoparticles was prepared by introducing a fluorescent dye (Nile red, 515-560 nm excitation; > 590 nm emission) in the same manner as in Preparation Example 5. Then, an O/W emulsion composition was prepared in the same manner as in Example 6. The inner phase of the emulsion composition prepared by introducing the fluorescent dye was observed with a microscope.

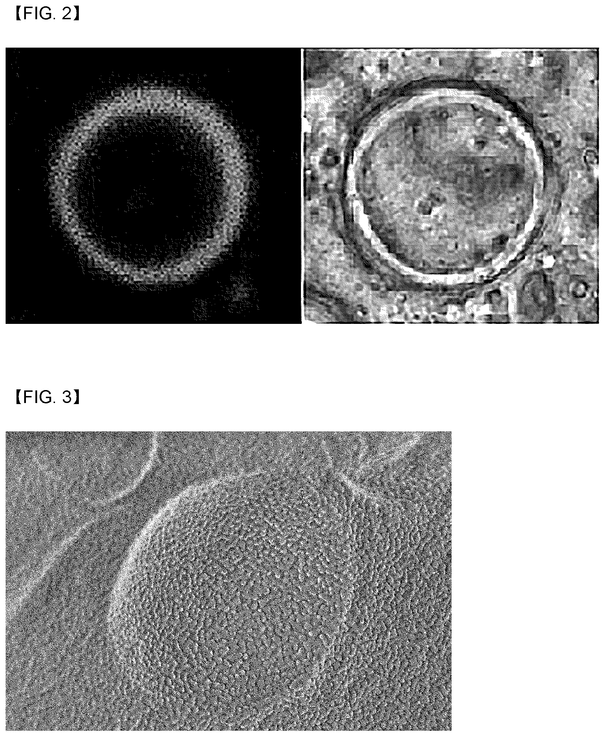

FIG. 2 shows laser confocal microscopic (VIVASCOPE 1500) images. It was confirmed that the fluorescent dye-supported interfacial particles, i.e. organic nanoparticles, were located at the interface between the inner phase and the outer phase. Thus, it was confirmed that the emulsion composition according to an aspect of the present disclosure can form a stable inner phase by stabilizing the interface between the inner phase and the outer phase without using a surfactant.

### Test Example 3. Microscopic observation and rheological analysis of emulsion composition

A conventional O/W emulsion composition was prepared by a conventional method according to the composition (wt%) described in Table 6 and used for the following test.

**[Table 6]**

| INCI | Content |
|---|---|
| Glyceryl stearate * PEG-1 00stearate | 5.00 |
| Squalane | 7.00 |
| Diisostearyl malate | 5.00 |
| Dimethicone | 4.00 |
| Purified water | To 100 |
| Disodium EDTA | 0.05 |
| Glycerin | 0.50 |
| Glyceryl caprylate | 0.10 |
| Ethylhexyl glycerin | 0.05 |
| Behenyl alcohol | 2.00 |
| Fragrance | 0.05 |
| Purified water * sorbitan isostearate * polyacrylate-13* polyisobutene * polysorbate 20 | 0.43 |

In addition, a Pickering emulsion composition was prepared by a conventional method according to the composition (wt%) described in Table 7 and used for the following test.

**[Table 7]**

| INCI | Content |
|---|---|
| Purified water | To 100 |
| Disodium EDTA | 0.05 |
| Butylene glycol | 10.00 |
| Phenoxyethanol | 0.20 |
| Ethylhexyl glycerin | 0.05 |
| C12-15 alkyl benzoate | 5.00 |
| Ethylhexyl methoxycinnamate | 3.00 |
| Bis-ethylhexyloxyphenol methoxyphenyl triazine | 3.00 |
| Cyclopentasiloxane * cyclohexasiloxane | 5.00 |
| Silica | 2.00 |
| Titanium dioxide * stearic acid * alumina | 2.00 |
| Fragrance | 0.25 |
| Purified water * sorbitan isostearate * polyacrylate-13 * polyisobutene * polysorbate 20 | 1.00 |

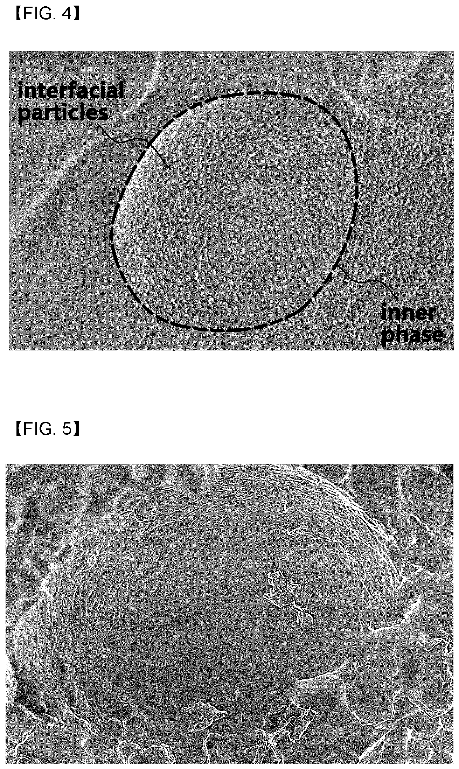

The O/W emulsion composition prepared with the composition described in Table 6 and the emulsion composition prepared in Example 5 were observed with a cryo-field emission scanning electron microscope (Cryo-FE-SEM, Nova SEM, FEI Company) and the surface of the inner phase was compared. As a result, it was confirmed that the emulsion composition prepared in Example 5 had a plurality of interfacial particles with a millet-like shape located on the surface of the inner phase (see FIGS. 3 and 4). In contrast, the inner phase of the conventional O/W emulsion composition formed by using a surfactant had a smooth surface as shown in FIG. 5. That is to say, it had a totally different structure from that of the emulsion composition according to an aspect of the present disclosure.

In general, organic particles such as nanoemulsion particles, solid lipid nanoparticles, liposomes, polymersomes, etc. have been used merely as carriers for active ingredients. An emulsion composition formed by using a surfactant shows a smooth surface of the inner phase as shown in FIG. 5 even when it includes organic particles, unlike the surface of the inner phase in which a plurality of organic particles are located at the interface between the inner phase and the outer phase as shown in FIG. 3. Therefore, it can be seen that the inner phase of the emulsion composition including the organic particles is not formed by the action of the organic particle as interfacial particles. The emulsion composition according to an aspect of the present disclosure is distinguished from the conventional emulsion composition including organic particles as carriers for active ingredients.

In addition, for the conventional emulsion composition formed by using a surfactant, the interface between the inner phase and the outer phase becomes unstable and fusion of the inner phase occurs as the size of the inner phase is increased, even when organic particles are included. Therefore, there is a limitation in increasing the size of the inner phase and providing the unique feeling of use of the emulsion composition according to an aspect of the present disclosure.

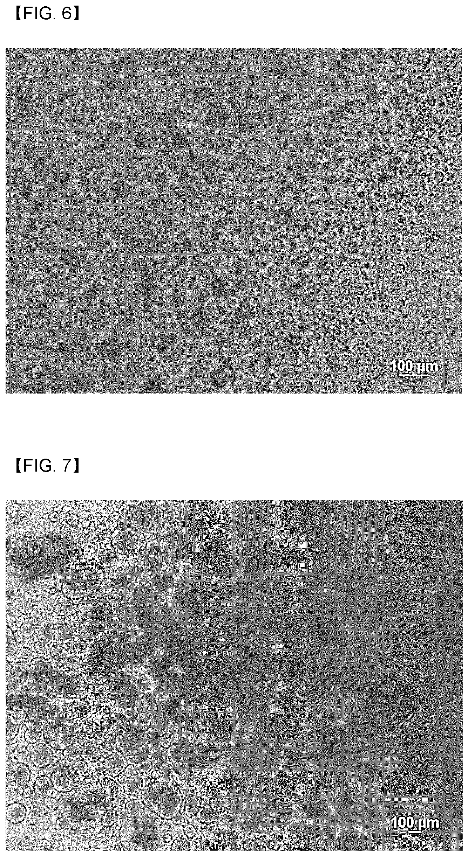

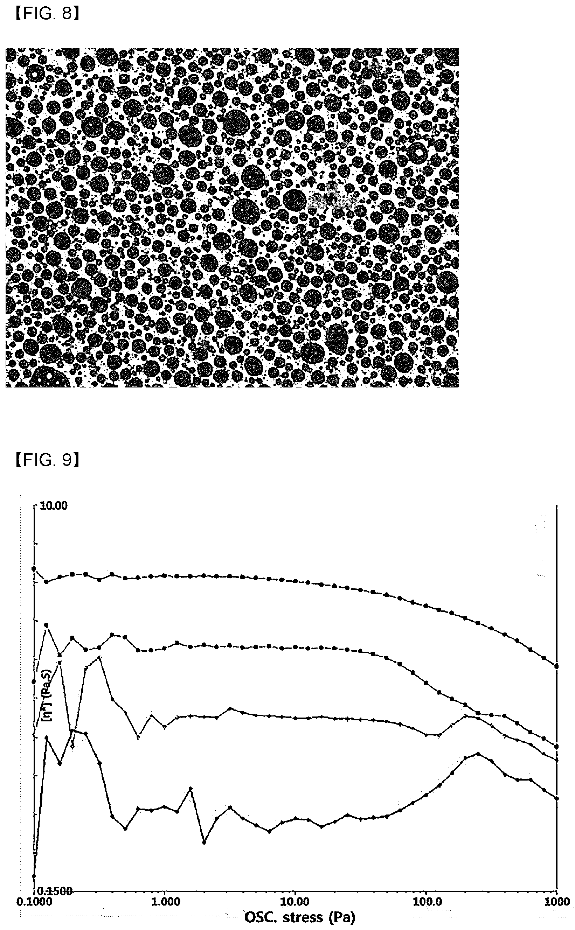

The emulsion compositions prepared in Examples 1 and 2 and the Pickering emulsion composition prepared with the composition described in Table 7 were observed with an optical microscope (DSX110, Olympus). The result is shown in FIGS. 6-8. In general, during observation with an optical microscope, use of a cover glass is determined as desired. In this test example, a different image was obtained for the Pickering emulsion composition from those of the emulsion compositions of Examples 1 and 2 because it was observed using a cover glass (see FIG. 8). FIGS. 7 and 8 show black-colored portions due to the use of the inorganic particles. For the emulsion composition of Example 2 (see FIG. 7), a larger area of black-colored portions was observed as compared to the emulsion composition of Example 1 (see FIG. 6) due to a relatively higher content of the inorganic particles.

The conventional O/W emulsion composition prepared with the composition described in Table 6, the Pickering emulsion composition prepared with the composition described in Table 7 and the emulsion compositions of Examples 6 and 7 were subjected to rheology measurement (rheometer (AR2000), measurement condition: osc.stress (Pa) 0.1-1000 @ frequency 50 Hz, room temperature). The result is shown in FIG. 9.

It was confirmed that the conventional O/W emulsion composition undergoes structural collapse at a shear stress of about 100 Pa and shows spreadability upon application to skin. In contrast, the emulsion compositions of Examples 6 and 7 showed a yield stress value at 0.1-1.0 Pa. This means that they can provide initial crumbling feeling, i.e. initial water burst feeling, unlike other formulations. Whereas the emulsion compositions of Examples 6 and 7 showed zero shear viscosity value, i.e. an initial viscosity value similar to the initial viscosity level of the conventional O/W emulsion composition, and thus provided a feeling of thickness similar to the conventional O/W emulsion composition upon the initial pickup and skin application, they underwent structural collapse at a very low shear stress of 0.1-0.2 Pa and showed dramatic phase change on the skin. Such a structural collapse provides a feeling of fast change from a feeling of use of heavy cream into a feeling of use, e.g., spreadability, of soft lotion. Such phase change (phase transition) is recognized by the user as a unique feeling of use. In addition, the emulsion compositions of Examples 6 and 7 showed increase in viscosity in the later stage, like the shear-thickening phenomenon. This is because the characteristics of the interfacial particles appear in the later stage. Therefore, the emulsion composition according to an aspect of the present disclosure can provide smooth finish feeling as well as the effect of improving skin roughness and moisturization by virtue of the application of the interfacial particles.

As described above, the emulsion compositions of Examples 6 and 7 are distinguished from the conventional O/W emulsion composition or the Pickering emulsion composition in that they undergo shear-thinning rapidly at an initial low shear stress to maximize spreadability and provide soft finish feeling by forming a moisturizing film on the skin by the interfacial particles in the vicinity of 100-1000 Pa where moisture and active ingredients are absorbed into the skin and the shear stress is further increased.

### Test Example 4. Confirmation of formulation stability of emulsion composition

### (1) Confirmation of formulation stability after freeze-drying

The emulsion compositions prepared in Examples 1-7 and Comparative Example 1 and the conventional O/W emulsion composition prepared in Test Example 3 with the composition described in Table 6 were freeze-dried and their formulation stability was investigated.

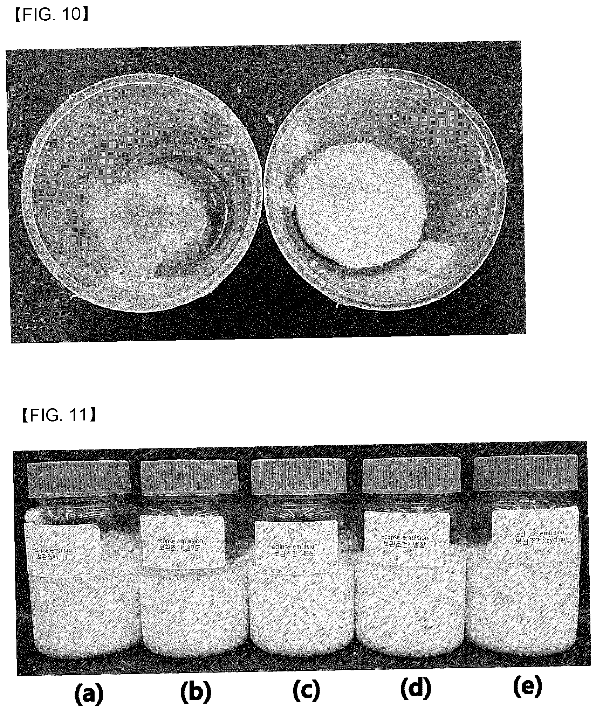

Freeze-drying is a kind of drying methods, and means a method which includes freezing a material and reducing partial pressure of water vapor so that a dried product may be obtained through a sublimation process whereby ice is converted directly into vapor. A sample was frozen at a temperature of -80 °C or lower using a freezer and then transferred to a drier to carry out sublimation. As a result, it was observed that the emulsion compositions of Examples 1-7 showed no oil separation, whereas the conventional O/W emulsion compositions of Comparative Example 1 and Test Example 3 showed oil separation. FIG. 10 compares the conventional O/W emulsion composition of Test Example 3 (left image) and the emulsion composition of Example 6 (right image).

Freeze-drying is advantageous in that active ingredients may be stabilized and applied to various types of products depending on the purpose of use through reprocessing into powder form and rehydration upon use. The conventional O/W emulsion composition such as that of Test Example 3 shows shrinking and deformation in appearance during freeze-drying, which cause oil oozing, and thus cannot be provided as a freeze-dried formulation. In contrast, the emulsion composition according to an aspect of the present disclosure maintains the inner phase structure well without oil separation even during freeze-drying, and thus has excellent formulation stability. In the case of the conventional O/W emulsion composition, further attempt is required to retain the inner phase structure well, and thus an undesired feeling of use appears often. However, when an emulsion composition is formed by using interfacial particles like the emulsion composition according to an aspect of the present disclosure, the inner phase structure is maintained well due to the packing of the interfacial particles. Thus, superior formulation stability and feeling of use can be achieved without using any additional material.

### (2) Confirmation of formulation stability depending on temperature change

The emulsion composition of Example 6 was observed in terms of formulation stability depending on temperature change. The result is shown in FIG. 11. It was confirmed that the emulsion composition maintains formulation stability when stored at room temperature, 37 °C, 45 °C and under a refrigerated condition individually for 4 weeks. In addition, it was confirmed that the emulsion composition maintains formulation stability without oil separation when stored in a cycling mode for total 4 weeks with a period of 12 hours by applying temperature change from 40 °C to -10 °C and then from -10 °C to 40 °C.

In addition, the formulation stability of the emulsion composition of Example 6 was observed by measuring viscosity with the lapse of time. The result is shown in Table 8. The viscosity was measured for a sample stored at 30 °C using a Brookfield viscometer (LVDV-2+P) (S64 spindle, 12 rpm, measured for 2 minutes). As a result, it was confirmed that the emulsion composition according to an aspect of the present disclosure has superior formulation stability.

**[Table 8]**

| Change with time (viscosity) | |
|---|---|
| | Example 6 |
| Right after preparation | 12500 cps |
| After 1 week | 14800 cps |
| After 2 weeks | 15600 cps |
| After 3 weeks | 14500 cps |
| After 4 weeks | 15300 cps |

### Test Example 5. Skin delivery effect of active ingredient

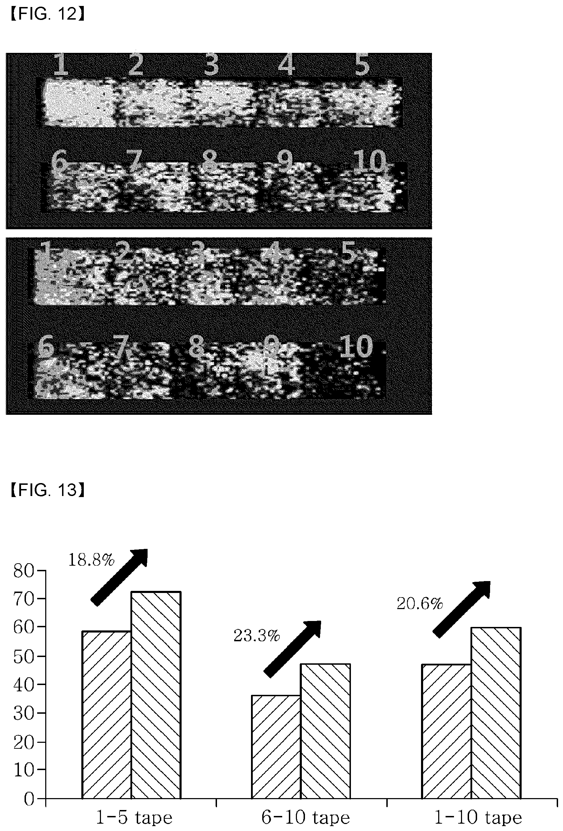

The emulsion composition prepared in Example 7 was used to determine the skin delivery effect of tocopherol as an active ingredient contained in interfacial particles. The result is shown in FIG. 12. The skin delivery effect of the active ingredient was compared with that of a conventional O/W emulsion composition prepared by adding 0.5 wt% of tocopherol to the O/W emulsion composition of Test Example 3 having the composition described in Table 6.

To determine the skin delivery effect of the active ingredient, each composition was applied to skin. 3 hours later, the composition remaining on the surface was wiped out and taping was carried out with 10 sheets of a skin keratin-collecting stripping tape (D-Squame Stripping Discs D101, Cuderm). After that, the active ingredient tocopherol attached to the tape was imaged through DESI-MS imaging technology (QTOF-MS (Q-time of flight mass spectrometer)) to determine the skin delivery effect.

As a result, it was confirmed that the emulsion composition of Example 7 delivered the active ingredient more deeply into the skin, as compared to the conventional O/W emulsion composition. In FIG. 12, the light-colored portion represents the active ingredient.

### Test Example 6. Skin absorbability of active ingredient

The emulsion composition of Example 7 was used to determine the skin absorbability of tocopherol as an active ingredient contained in interfacial particles. The result is shown in FIG. 13. The skin absorbability of the active ingredient was compared with that of a conventional O/W emulsion composition prepared by adding 0.5 wt% of tocopherol to the O/W emulsion composition of Test Example 3 having the composition described in Table 6.

To determine the skin absorbability of the active ingredient, each composition was applied to skin. 3 hours later, the composition remaining on the surface was wiped out and taping was carried out with 10 sheets of a skin keratin-collecting stripping tape (D-Squame Stripping Discs D101, Cuderm). Skin absorption was investigated by carrying out HPLC analysis to analyze the active ingredient tocopherol attached to the tape (Waters e2965, SunFire Silica column (4.6 x 250 mm x 5 µm), hexane : isopropanol = 99 : 1, flow rate 0.7 mL/min, 325 nm).

As a result, it was confirmed that the emulsion composition of Example 7 provides high skin absorbability of the active ingredient, as compared to the conventional O/W emulsion composition. In FIG. 13, 1-5 tape, 6-10 tape and 1-10 tape represent the average value of Nos. 1-5 tapes, average value of Nos. 6-10 tapes and average value of Nos. 1-10 tapes, respectively. And, the y-axis represents tocopherol absorption (%).

### Test Example 7. Investigation of change in skin texture and water content after application of emulsion composition

The emulsion composition of Example 6 was applied to the skin and the change in skin texture and water content was observed.

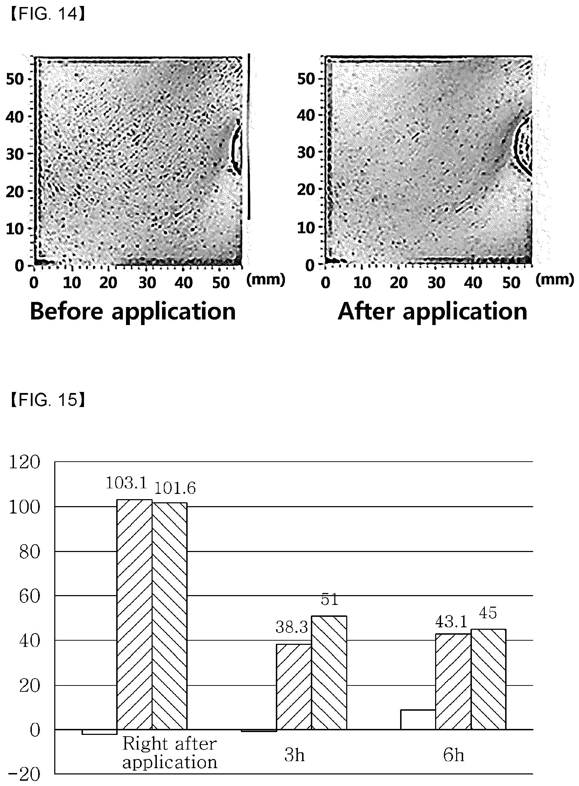

When the emulsion composition of Example 6 was applied to skin, skin roughness was decreased by about 14.4% as compared to before the application (see FIG. 14). In addition, when the change in water content was compared to the non-application test group and the composition application test group using the conventional O/W emulsion composition of Test Example 3 having the composition described in Table 6, it was confirmed that the emulsion composition of Example 6 allows the skin to retain water for a longer time (see, FIG. 15). This is because the interfacial particles are packed in the skin surface while they burst upon application to skin, thereby preventing water loss and making the skin texture smooth.

### Test Example 8. Investigation of change in skin under dry environment after application of emulsion composition

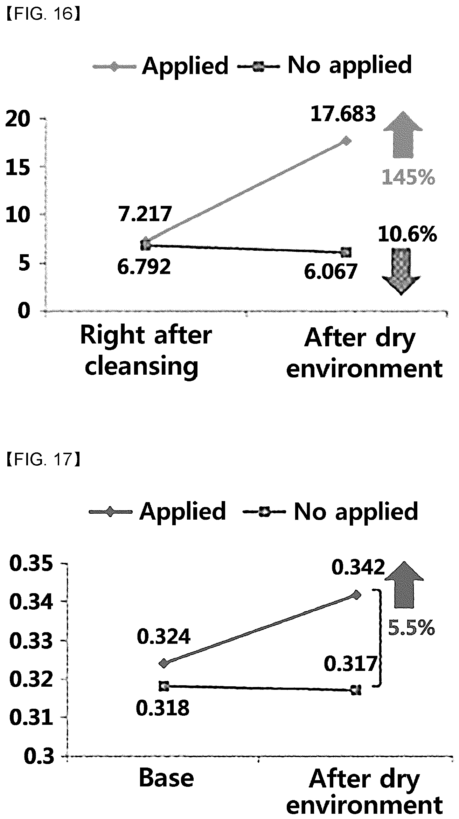

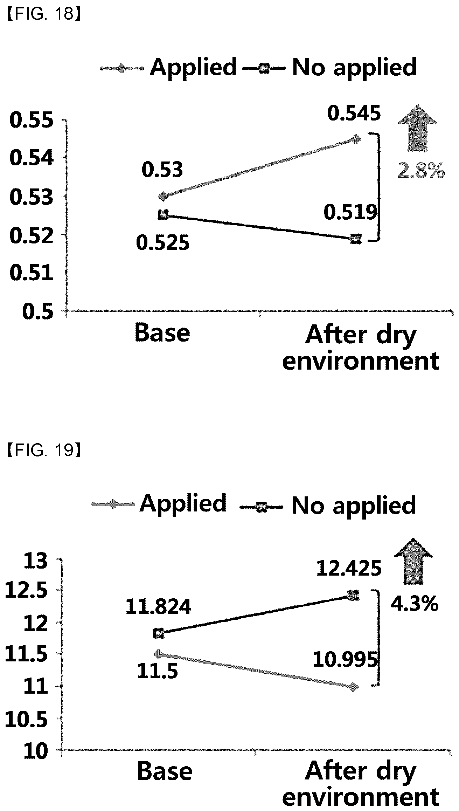

The emulsion composition of Example 7 was applied to skin and the change in skin water content, eye rim skin flexibility, eye rim skin elasticity and eye rim wrinkles under dry environment was investigated. The result is shown in FIGS. 16-19. The dry environment was created by waiting under the condition of a relative humidity below 35% for 1 hour.

As a result, it was confirmed that the application of the emulsion composition according to Example 7 provides effects of enhancing skin water content, eye rim skin flexibility and eye rim skin elasticity and preventing eye rim wrinkles even under dry environment, unlike the test group to which no composition was applied.

### Test Example 9. Effect of reducing skin irritation caused by active ingredient 1

An O/W emulsion composition was prepared with the composition (wt%) described in Table 9 in the same manner as described above to determine the skin irritation level and potency of retinol.

**[Table 9]**

| | Ex. 8 | Ex. 9 | Ex. 10 | Ex. 11 | Comp. Ex. 2 | Comp. Ex. 3 | Comp. Ex. 4 |
|---|---|---|---|---|---|---|---|
| Hydrogenated C₆₋₁₄ olefin polymer | 3 | 3 | 3 | 3 | 3 | 3 | 3 |
| Squalane | 2 | 2 | 2 | 2 | 2 | 2 | 2 |
| Dimethicone | 5 | 5 | 5 | 5 | 5 | 5 | 5 |
| Shea butter | 2 | 2 | 2 | 2 | 2 | 2 | 2 |
| Soybean oil * retinol * BHT (dibutylhydroxytoluene) | 3 | | 1 | | | 3 | 1 |
| Glyceryl stearate * PEG-100 stearate | | | | | | 1.5 | 1.5 |
| Water | To 100 | To 100 | To 100 | To 100 | To 100 | To 100 | To 100 |
| Water-dispersible colloidal solution of Preparation Example 2 | 10 | | 10 | | 10 | | |
| Water-dispersible colloidal solution of Preparation Example 3 (soybean oil * retinol * BHT 10 wt%) | | | | 10 | | | |
| Water-dispersible colloidal solution of Preparation Example 3 (soybean oil * retinol * BHT 30 wt%) | | 10 | | | | | |
| 1,2-Hexanediol | 1 | 1 | 1 | 1 | 1 | 1 | 1 |
| Ethylhexylglycerin | 0.05 | 0.0 5 | 0.05 | 0.05 | 0.05 | 0.05 | 0.05 |
| Carbomer | 0.2 | 0.2 | 0.2 | 0.2 | 0.2 | 0.2 | 0.2 |
| Tromethamine | 0.1 | 0.1 | 0.1 | 0.1 | 0.1 | 0.1 | 0.1 |
| Skin irritation | No | No | No | No | No | Light | No |

The O/W emulsion compositions of Examples 8-11 and Comparative Example 2 were prepared using a water-dispersible colloidal solution, and the conventional O/W emulsion compositions of Comparative Examples 3 and 4 were prepared using a surfactant. It was confirmed that the emulsion compositions of Examples 8-11 and Comparative Example 2 form stable emulsion systems owing to the organic particles regardless of the presence of retinol as an active ingredient. In Examples 8 and 10, retinol was supported in the inner phase, i.e. emulsion particles, whereas retinol was supported in interfacial particles, i.e. organic particles, in Examples 9 and 11.

Skin irritation was tested as follows. Each test sample was attached to the forearm site of 22 subjects in the form of a closed patch by using the IQ Chamber^{™} system 6 times for 24 hours. The response was checked as follows according to the CTFA guideline (1981) and the criteria defined by Frosch and Kligman (1979) up to the application finishing point or maximum response point.
Grade 0: No response
Grade 1: light erythema, macule or heat emission
Grade 1: moderate erythema
Grade 3: strong erythema accompanied by edema
Grade 4: strong erythema accompanied by edema and blister

Retinol has an advantage of an excellent effect of improving skin wrinkles, but is problematic in that it causes skin irritation and shows low stability depending on the user's skin type. Particularly, in a formulation containing a surfactant that may cause skin irritation, the problem of skin irritation may be aggravated due to the surfactant. As can be seen from Comparative Examples 3 and 4, when retinol content is increased, skin irritation is also increased. In contrast, as can be seen from Examples 8-11, when an emulsion system was formed by using organic particles or by using retinol-supported organic particles, it was possible to significantly reduce the skin irritation caused by retinol. The emulsion composition of Comparative Example 2, which contains neither retinol nor a surfactant, caused no skin irritation.

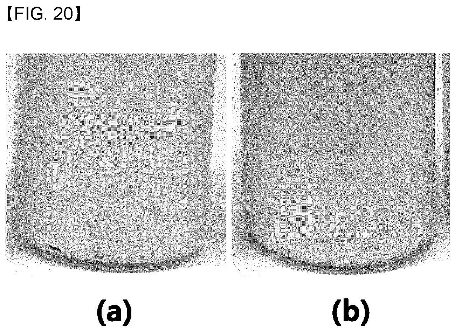

In addition, each of the emulsion compositions of Examples 8-11 was stored at 40 °C for 4 weeks and the potency of retinol contained in the emulsion composition was investigated. Retinol content was analyzed by high-performance liquid chromatography (HPLC). It was confirmed that the stability of retinol in the formulation was 93% or higher. In addition, it was confirmed that the content causes no significant discoloration even after storage 40 °C for 4 weeks (see, FIG. 20).

### Test Example 10. Effect of reducing skin irritation caused by active ingredient 2

An emulsion composition was prepared according to the composition (wt%) described in Table 11 in the same manner as described above. The skin irritation level was determined in the same manner as Test Example 9.

**[Table 10]**

| | Comp. Ex. 5 | Comp. Ex. 6 | Comp. Ex. 7 |
|---|---|---|---|
| Squalane | To 100 | To 100 | To 100 |
| Bakuchiol | 2.5 | 1 | 0.5 |
| Skin irritation | Light | Light | Light |

**[Table 11]**

| | Ex. 12 | Ex. 13 | Ex. 14 | Ex. 15 | Ex. 16 | Ex. 17 | Comp . Ex. 8 | Comp . Ex. 9 | Comp. Ex. 10 | Comp. Ex. 11 |
|---|---|---|---|---|---|---|---|---|---|---|
| Hydrogenated C₆₋₁₄ olefin polymer | 3 | 3 | 3 | 3 | 3 | 3 | 3 | 3 | 3 | 3 |
| Squalane | 2 | 2 | 2 | 2 | 2 | 2 | 2 | 2 | 2 | 2 |
| Cyclopentasiloxane * cyclohexasiloxane | 5 | 5 | 5 | 5 | 5 | 5 | 5 | 5 | 5 | 5 |
| Shea butter | 1.5 | 1.5 | 1.5 | 1.5 | 1.5 | 1.5 | 1.5 | 1.5 | 1.5 | 1.5 |
| Bakuchiol | 2.5 | | 1 | | 0.5 | | | 2.5 | 1 | 0.5 |
| Glyceryl stearate * PEG-100 stearate | | | | | | | | 1.5 | 1.5 | 1.5 |
| Water | To 100 | To 100 | To 100 | To 100 | To 100 | To 100 | To 100 | To 100 | To 100 | To 100 |
| Water-dispersible colloidal solution of Preparation Example 2 | 10 | | 10 | | 10 | | 10 | | | |
| Water-dispersible colloidal solution of Preparation Example 4 (bakuchiol 5 wt%) | | | | | | 10 | | | | |
| Water-dispersible colloidal solution of Preparation Example 4 (bakuchiol 10 wt%) | | | | 10 | | | | | | |
| Water-dispersible colloidal solution of Preparation Example 4 (bakuchiol 25 wt%) | | 10 | | | | | | | | |
| 1,2-Hexanediol | 1 | 1 | 1 | 1 | 1 | 1 | 1 | 1 | 1 | 1 |
| Ethylhexylglycerin | 0.05 | 0.05 | 0.05 | 0.05 | 0.05 | 0.05 | 0.05 | 0.05 | 0.05 | 0.05 |
| Carbo mer | 0.2 | 0.2 | 0.2 | 0.2 | 0.2 | 0.2 | 0.2 | 0.2 | 0.2 | 0.2 |
| Tromethamine | 0.1 | 0.1 | 0.1 | 0.1 | 0.1 | 0.1 | 0.1 | 0.1 | 0.1 | 0.1 |
| Skin irritation | No | No | No | No | No | No | No | Light | Light | Light |

The O/W emulsion compositions of Examples 12-17 and Comparative Example 8 were prepared using a water-dispersible colloidal solution, and the conventional O/W emulsion compositions of Comparative Examples 9-11 were prepared using a surfactant. It was confirmed that the emulsion compositions of Examples 12-17 and Comparative Example 8 form stable emulsion systems owing to organic particles regardless of the presence of bakuchiol as an active ingredient. In Examples 12, 14 and 16, bakuchiol was supported in the inner phase, i.e. emulsion particles, at contents of 2.5 wt%, 1 wt% and 0.5 wt%, respectively. In Example 13, 15 and 17, bakuchiol was supported in interfacial particles, i.e. organic particles, at contents of 2.5 wt%, 1 wt% and 0.5 wt%, respectively.

In order to investigate skin irritation caused by the bakuchiol active ingredient, the oil-soluble ingredient bakuchiol was introduced to squalane causing no skin irritation. As a result, it was confirmed that skin irritation occurred in all of the test groups containing bakuchiol of 0.5 wt% or higher (see Table 10). In addition, as can be seen from Comparative Examples 9-11, the conventional emulsion compositions containing bakuchiol also caused skin irritation. In contrast, as can be seen from the emulsion compositions of Examples 12-17, when the emulsion systems were formed using organic particles or by bakuchiol-supported organic particles, it was possible to reduce the skin irritation caused by bakuchiol significantly. The emulsion composition of Comparative Example 8, which contains neither bakuchiol nor a surfactant, caused no skin irritation.

While the present disclosure has been described with respect to the specific embodiments, it will be apparent to those skilled in the art that these exemplary embodiments are for illustrative purposes only and are not intended to limit the scope of the present disclosure. Therefore, the scope of the present disclosure is defined only by the following claims and equivalents thereof.

## Claims

1. An emulsion composition comprising:
an inner phase;
an outer phase; and
a plurality of interfacial particles which surround a surface of the inner phase so as to form an interface,
wherein the interfacial particles have a smaller particle size than a size of the inner phase, and
the interfacial particles comprise organic particles.

2. The emulsion composition according to claim 1, wherein the inner phase has a mean size of 1-50 µm.

3. The emulsion composition according to claim 1, wherein the interfacial particles have a mean particle size corresponding to 1/5,000-1/2 of the mean size of the inner phase.

4. The emulsion composition according to claim 1, wherein the interfacial particles have a mean particle size of 10 nm or more and less than 1 µm.

5. The emulsion composition according to claim 1, wherein the interfacial particles are continuously located on the surface of the inner phase to form the interface between the inner phase and the outer phase.

6. The emulsion composition according to claim 1, wherein the interfacial particles further comprise inorganic particles.

7. The emulsion composition according to claim 1, wherein the emulsion composition comprises a colloidal solution as an emulsifier,
the colloidal solution comprises a dispersive medium and organic particles dispersed in the dispersive medium,
the dispersive medium of the colloidal solution is miscible with the outer phase of the emulsion composition and immiscible with the inner phase of the emulsion composition, and
after emulsification with the emulsifier, the dispersive medium is incorporated into the outer phase, and the organic particles dispersed in the dispersive medium surround the surface of the inner phase.

8. The emulsion composition according to claim 7, wherein the colloidal solution is present in an amount of 7-30 wt% based on the total weight of the emulsion composition.

9. The emulsion composition according to claim 1, wherein the organic particles are dispersed only in the outer phase of the emulsion composition and not in the inner phase of the emulsion composition.

10. The emulsion composition according to claim 1, wherein the organic particles are selected from a group consisting of nanoemulsion particles, solid lipid nanoparticles (SLN), liposomes and polymersomes.

11. The emulsion composition according to claim 1, wherein the organic particles comprise an active ingredient.

12. The emulsion composition according to claim 11, wherein the active ingredient comprises bakuchiol or retinol.

13. The emulsion composition according to claim 11, wherein the active ingredient is supported on the organic particles.

14. The emulsion composition according to claim 11, wherein the active ingredient is present in an amount of 0.0001-10 wt% based on the total weight of the emulsion composition.

15. The emulsion composition according to claim 1, wherein the emulsion composition is a cosmetic composition.

16. The emulsion composition according to claim 1, wherein the emulsion composition further comprises a surfactant.

17. The emulsion composition according to claim 1, wherein the emulsion composition further comprises a thickener.

18. The emulsion composition according to claim 1, wherein the emulsion composition exhibits a yield stress of 0.1-1.0 Pa when viscosity depending on shear stress is measured.

19. The emulsion composition according to claim 18, wherein the emulsion composition exhibits a decrease in viscosity by 30% or more out of the yield stress of 0.1-1.0 Pa.

20. The emulsion composition according to claim 1, wherein the emulsion composition exhibits an interval in which viscosity is increased as shear stress is increased, in at least a part of a shear stress interval of 100-1,000 Pa, when viscosity depending on shear stress is measured.

21. The emulsion composition according to claim 1, wherein the emulsion composition is a freeze-dried formulation.

22. A method for preparing the emulsion composition according to any of claims 1 to 21, comprising:
preparing a colloidal solution comprising a dispersive medium and organic particles dispersed in the dispersive medium;
preparing an aqueous phase part;
preparing an oil phase part; and
mixing the colloidal solution, the aqueous phase part and the oil phase part to prepare an emulsion composition comprising an inner phase, an outer phase and a plurality of interfacial particles which surround a surface of the inner phase so as to form an interface,
wherein the interfacial particles have a smaller particle size than a size of the inner phase, and the interfacial particles comprise organic particles.

23. The method according to claim 22, which further comprises adding inorganic particles to the oil phase part or the emulsion composition.
